# EUROPÄISCHE PATENTSCHRIFT

(11) **EP 1 206 235 B1**
(45) Veröffentlichungstag und Bekanntmachung des Hinweises auf die Patenterteilung: **29.09.2004**
(21) Anmeldenummer: 00958316.2
(22) Anmeldetag: 28.07.2000
(51) Int. Cl.: A61K 6/00, A61K 6/083, A61K 6/087

(54) **VERWENDUNG EINER ZUSAMMENSETZUNG ZUR HERSTELLUNG VON ADHÄSIVSYSTEMEN**
USE OF A COMPOSITION FOR THE MANUFACTURING OF ADHESIVE SYSTEMS
UTILISATION D'UNE COMPOSITION POUR LA PREPARATION DE SYSTEMES ADHESIFS

(30) Priorität: 06.08.1999 DE 19937092
(43) Veröffentlichungstag der Anmeldung: 22.05.2002
(73) Patentinhaber: 3M ESPE AG, 82229 Seefeld (DE)
(72) Erfinder: LUCHTERHANDT, Thomas, D-86926 Greifenberg (DE); GRUPP, Hendrik, M., D-86919 Utting (DE); GUGGENBERGER, Rainer, D-82211 Herrsching (DE)
(74) Vertreter: Brem, Roland
(86) Internationale Anmeldenummer: PCT/EP2000/007323
(87) Internationale Veröffentlichungsnummer: WO 2001/010389

(56) Entgegenhaltungen:
- EP-A- 0 712 622
- EP-A- 0 897 710
- WO-A-00/56800
- WO-A-98/46197
- DE-A- 19 648 283

## Beschreibung

Die vorliegende Erfindung betrifft die Verwendung einer radikalisch polymerisierbaren Zusammensetzung. Zur Herstellung von Adhäsivsystemen zur Befestigung von nur oder auch kationisch polymerisierbaren Materialien, auf Wasser enthaltenden Hartgewebe.

In polymerisierbaren Dentalmassen wurden bislang vorwiegend Methacrylat- und Acrylatmonomere verwendet. Besondere Aufmerksamkeit verdient das von Bowen beschriebene 2,2-Bis[4-(2-hydroxy-3-methacryloyloxypropoxy)phenyl]-propan (Bis-GMA) [US-A-3 066 112]. Mischungen dieses Methacrylats mit Triethylenglykoldimethacrylat dienen auch heute noch als Standardrezeptur für dentale plastische Direkt-Füllungswerkstoffe. Auch Methacrylderivate des zweifach formylierten Bis-(hydroxymethyl)tricyclo[5.2.1.0^{2,6}]decans haben sich als Monomere für Dentalcomposite bewährt [W. Gruber et al., DE-A-27 14 538; W. Schmitt et al., DE-A-28 16 823; J. Reiners et al., EP-A-0 261 520]. Ein wesentlicher Nachteil dieser Dentalmassen ist aber der durch die Polymerisation auftretende hohe Volumenschrumpf. Dieser kann beispielsweise durch den Einsatz von ringöffnenden Monomeren, wie den kationisch härtenden Epoxiden, minimiert werden.

Über kationisch härtbare Epoxidmassen für dentale Anwendungen ist nur wenig bekannt. Die US-A-5 556 896 beschreibt epoxidhaltige Massen, die notwendigerweise als schrumpfkompensierende Monomere Spiroorthocarbonate enthalten müssen. Bowen beschreibt eine Masse, enthaltend Quarzsand und ein aliphatisches Diepoxid (Bisphenol-A-diglycidylether), die im ausgehärteten Zustand angeblich gute Stabilität im Mundmilieu aufweist [J. Dent. Res. 35, 1956, 360-379]. Die AT-A-204 687 beschreibt Epoxid-Dentalmassen auf Basis von Bisphenol-A, die mittels Lewis-Säure-Katalysatoren ausgehärtet werden. Die Schriften DE-A-196 48 283, WO-96/13538 und WO-95/30402 beschreiben ebenfalls polymerisierbare Dentalmassen auf Basis von Epoxiden und deren Verwendung.

Obwohl es umfangreiche Erfahrungen mit Epoxiden und cycloaliphatischen Epoxiden gibt (US-A-2 716 123, US-A-2 750 395, US-A-2 863 881, US-A-3 187 018), sind solche Monomere und daraus formulierte kationisch polymerisierbare Massen mit den für dentale Anwendungen notwendigen Eigenschaften zu keinem Zeitpunkt kommerziell verfügbar gewesen.

Ursache dafür ist die Tatsache, dass die Aushärtung dieser kationisch polymerisierenden Massen durch Wasser inhibiert wird und die Zahnhartsubstanz, beispielsweise im Dentin, ca. 11 bis 16 Gewichtsprozent Wasser enthält (G.-H. Schumacher et. al., Anatomie und Biochemie der Zähne, Gustav Fischer Verlag, 1990, 4. Auflage).

So ist es leicht zu erklären, dass beispielsweise Formulierungen von dentalen Füllungsmaterialien auf Epoxidbasis, im Gegensatz zu Formulierungen auf (Meth)-Acrylatbasis, keine Eigenhaftung auf Dentin zeigen können und so die Verwendung solcher Materialien nicht möglich war.

Zur verbesserten Befestigung von dentalen Füllungsmaterialien auf (Meth)-Acrylatbasis - also radikalisch härtenden Systemen - werden sog. Adhäsivsysteme verwendet.

Die Qualität dieser Adhäsivsysteme spiegelt sich in den folgenden Kriterien wieder:
- Vollständige Haftung an der Zahnhartsubstanz ohne Fehlstellen ("Versiegelung"),
- vollständige Haftung an dem Füllungsmaterial,
- dauerhafter Verbund.

Da die Polymerisation von (Meth-)Acrylatsystemen aber über einen radikalischen Mechanismus abläuft, ist eine Haftung von kationisch polymerisierenden Zahnmaterialien auf solchen Adhäsivsystemen nicht zu erwarten. Auf dem Markt ist zu keiner Zeit ein dentales Adhäsivsystem für (auch) kationisch vernetzende Materialien kommerziell verfügbar gewesen.

Die DE-A-197 43 564 beschreibt zwar als strahlenhärtbare Haftvermittler - sog. Primer - Zusammensetzungen auf der Basis von lösungsmittelfreien, kationisch und/oder radikalisch härtbaren Vernetzungssystemen, doch werden diese nur zur Beschichtung von wasserfreien Materialien, beispielsweise Kunststoffen, wie Polyvinylidenchlorid (PVDC) oder Silikon, verwendet.

Die WO-98/47046 beschreibt photopolymerisierbare Mischungen auf Epoxidbasis, enthaltend ein Epoxidharz, ein lodoniumsalz, ein im sichtbaren Licht sensibles Übertragungsmolekül und einen Elektronendonor und deren Verwendung als dentales Adhäsivsystem. Es zeigt sich jedoch, dass mit solchen Mischungen auf der Zahnhartsubstanz keine Haftung zu kationisch härtenden Mischungen zu erzielen ist (siehe Vergleichsmischungen 1 bis 3).

Die WO-99/34766 führt aus, dass Zusammensetzungen mit einem hohen Anteil an kationisch härtbaren Gruppen nicht oder nur sehr schlecht auf Zahnhartgewebe haften. Zur Lösung des Problems wird vorgeschlagen entweder eine Hybrid-Zusammensetzung, enthaltend Bestandteile mit radikalisch und kationisch polymerisierbaren Gruppen, oder eine Zusammensetzung, die weitgehend frei ist von kationisch polymerisierbaren Gruppen, bereitzustellen.

Die WO 98/46197A beschreibt Dentalzusammensetzungen, die zu den verschiedensten Zwecken eingesetzt werden können, unter anderem als Füllmaterialien, Zemente oder auch dentale Klebstoffe. Es handelt sich um Zusammensetzungen auf der Basis von Monomeren mit polymerisierbaren Gruppen, die radikalisch oder kationisch gehärtet werden können. Als radikalisch härtbare Polymere werden unter anderem Acrylate genannt und als kationisch härtbare Polymere werden unter anderem expoxyhaltige Materialien erwähnt. Kombinationen von Adhäsivsystemen und Füllsystemen werden nicht angesprochen.

Die EP-A-0 712 622 betrifft dentale Adhäsivzusammensetzungen auf der Basis eines phosphorsäuregruppenhaltigen Monomeren, eines carbonsäuregruppenhaltigen Monomeren und Wasser als Hauptbestandteile, die radikalisch härten und sehr gut auf Zahnschmelz und Dentin haften sollen. Über die Natur der dentalen Füllmaterialien wird nichts gesagt.

Aufgabe der vorliegenden Erfindung ist es, Methoden zur Verfügung zu stellen, um entweder nur kationisch vernetzende Materialien oder radikalisch und kationisch vernetzende Materialien auf Wasser enthaltenden Hartgewebe, wie Zahn, zur Haftung zu bringen, wobei die Haftung vorzugsweise im wesentlichen gleichmäßig über die gesamte für die Haftung zur Verfügung stehende Fläche erfolgen soll.

Erfindungsgemäß wird diese Aufgabe gelöst durch Verwendung einer Zusammensetzung, die radikalisch polymerisierbar ist und 1 bis 30 Gew.-%, vorzugsweise nicht mehr als Gew.-20 % und in ganz bevorzugter Weise nicht mehr als Gew.-15 % eines reaktiven Lösungsmittels mit einem pKS-Werte kleiner oder gleich dem von Aceton enthält, zur Herstellung eines Adhäsivsystems zur Befestigung von Materialien, die nur oder auch kationisch polymerisierbar sind, auf Wasser enthaltenden Hartgewebe, wobei das Adhäsivsystem mindestens eine Komponente i), die befähigt ist, eine radikalische Reaktion zu starten, und eine Komponente ii), die radikalisch polymerisierbare Monomere enthält, die säurefunktionell sind oder Gruppen enthalten, die Säuren bilden können, enthält.

Überraschenderweise wurde gefunden, dass bei der Verwendung von radikalisch polymerisierenden Adhäsivsystemen und kationisch polymerisierbaren Dentalmaterialien eine gute Haftung auf Wasser enthaltenden Hartgewebe, wie Zahnhartsubstanz, erzielt werden kann, obwohl zwei völlig unterschiedliche Polymerisationsarten vorliegen.

Da die kationische Polymerisation über einen ionischen Mechanismus der Kettenfortpflanzung abläuft, gibt es der Theorie nach keine Möglichkeiten nichtradikalisch polymerisierende Monomere mit radikalisch wachsenden Ketten zu polymerisieren.

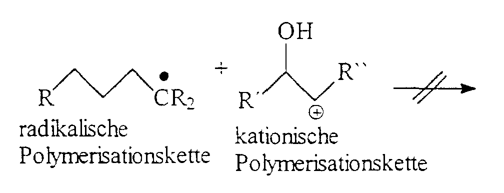

Auch sollte das im Hartgewebe enthaltende Wasser bzw. dem Adhäsivsystem zugesetzte reaktive Lösungsmittel die kationische Polymerisation stören, da es als Kettenabbruchmittel wirkt. Durch den fortwährenden Kettenabruch sollten sich somit viele kurze Ketten bilden, die den Aufbau eines polymeren Netzwerk behindern.

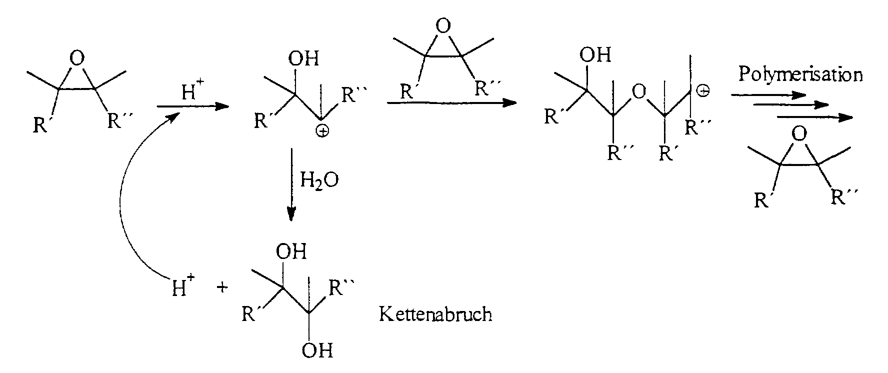

Völlig überraschend ist, dass trotz dem oben Gesagten kationisch polymerisierende Materialien auf der Wasser enthaltenden Zahnhartsubstanz oder auch auf radikalisch polymerisierenden, reaktive Lösungsmittel, enthaltenden Adhäsivsystemen zur Haftung gebracht werden können. Vorteilhaft ist ferner die im Vergleich zu üblichen Lösungsmitteln, wie CH₂Cl₂ oder Acetonitril, geringere Toxizität und die verminderte Flüchtigkeit, die es erlaubt, die adhäsive Mischung gleichmäßig aufzutragen bevor das Lösungsmittel verdampft. Vorteilhaft sind auch verbesserte Lösungseigenschaften, die den Einsatz einer großen Anzahl unterschiedlicher Monomere ermöglichen.

Im Folgenden wird die Erfindung näher beschrieben.

Die adhäsive Mischung, welche die beschriebenen Vorteile bei erfindungsgemäßer Verwendung aufweisen, enthält als Bestandteil i) vorzugsweise 0,01 bis 10 Gew.-%, insbesondere 0,1 bis 7 Gew.-%, und besonders bevorzugt 0,1 bis 5 Gew.-% eines Initiatorsystems, das befähigt, ist eine radikalische Polymerisation zu starten und als Bestandteil ii) vorzugsweise 90 bis 99,99 Gew.-%, insbesondere 93 bis 99,90 Gew.-% und besonders bevorzugt 95 bis 99,90 Gew.-% des radikalisch polymerisierbaren Materials.

Der Anteil des reaktiven Lösungsmittels liegt dabei im Bereich von 1 bis 30 Gew.-%, bevorzugt im Bereich von 5 bis 25 Gew.-% und ganz besonders bevorzugt im Bereich 8 bis 20 Gew.-%.

Reaktive Lösungsmittel sind solche mit aciden Protonen und einem pKS-Wert kleiner oder gleich dem von Aceton, wie Wasser, Methanol, Ethanol, n- und i-Propanol. In Versuchen hat sich gezeigt, dass solche Lösungsmittel insbesondere solche, die Hydroxylgruppen tragen, entgegen dem oben gesagten die Haftung zwischen kationischen Materialien und radikalisch polymerisierenden Materialien verbessern.

Die radikalbildenden Initiatoren, welche als Komponente i) in den Mischungen enthalten sein können, sind in der Literatur beschrieben (z. B. J.-P Fouassier, Photoinitiation, Photopolymerization and Photocuring, Hanser Publishers, Munich, Vienna, New York, 1995 oder auch J.-P Fouassier, J. F. Rabek (Hrsg.), Radiation Curing in Polymer Science and Technology, Vol. II, Elsevier Apllied Science, London, New York, 1993). Sie können durch UV- oder sichtbares Licht aktivierbare Substanzen sein, wie Benzoinalkylether, Benzilketale, Acylphosphinoxide oder aliphatische und aromatische 1,2-Diketonverbindungen, beispielsweise Campherchinon, wobei die Katalysatoraktivität durch Zusatz von Aktivatoren, wie tertiären Aminen, beispielsweise Dialkylamino-4-bensoesäureester, oder organischen Phosphiten, in an sich bekannter Weise beschleunigt werden kann.

Geeignete Initiatorsysteme zur Auslösung der radikalischen Polymerisation über einen Redox-Mechanismus sind beispielsweise die Systeme Peroxid/Amin, Peroxid/Barbitursäurederivate oder Peroxid/Säuren. Bei Verwendung solcher Initiatorsysteme ist es zweckmäßig, einen Initiator (z. B. Peroxid) und eine Katalysatorkomponente (z. B. Amin) getrennt bereitzuhalten. Die beiden Komponenten werden dann kurz vor ihrer Anwendung miteinander homogen vermischt.

Als Komponente ii) können die üblichen, in radikalisch härtenden Dentalmaterialien verwendeten Monomere eingesetzt werden, wobei die Komponente ii) 3 bis 100 Gew.-% radikalisch polymerisierbare Monomere enthalten muss, die säurefunktionell sind, oder solche Gruppen enthalten, die mit Wasser Säuren bilden können, wie beispielsweise Säurechloride oder Anhydride, wobei mit Säuren Mono-, Di- oder Polycarbonsäuren, mit folgenden Resten gemeint sind: C₁- bis C₂₅- Alkyl- oder -Cycloalkylreste ggf. mit N, O, S, Si, P oder Halogen substituiert, aromatische C₆ bis C₁₂-Reste oder heterocyclische C₃ bis C₁₂-Reste mit N, O, S, P und ggf. mit Halogen substituiert. Genauso können Säuren, wie die 4-Methacryloxy-ethyltrimelitsäure oder ihre Anhydride (Takeyama, M. et al., J.Jap.Soc. f. Dent. App. A. Mat. 19, 179 (1978)) oder die Umsetzungsprodukte von Trimellitsäurechloridanhydrid mit aminischen, thiolischen oder hydroxylischen (Meth-)Acrylsäureestern, wie beispielsweise 2-Hydroxyethylenmethacrylat (HEMA) oder Methacroyloxy-ethyl-o-phthalat, verwendet werden.

Andere bevorzugte Säuren sind ungesättigte organische Ester der Monofluorphosphonsäuren, wie sie in der US-A-3 997 504 beschrieben sind, ungesättigte organische Ester von Säuren des Phosphors, die Chlor oder Brom direkt am Phosphor gebunden enthalten, wie sie in der EP-A-0 058 483 beschrieben sind, ungesättigte organische Ester der Phosphorsäure, die als cyclische Pyrophosphate (Anhydride) vorliegen, wie sie in der DE-A-3 048 410 beschrieben sind und ungesättigte organische Ester von Phosphor- oder Phosphonsäuren, wie sie in den DE-A-2 711234 und DE-a-3 150 285 beschrieben sind. Genauso bevorzugt sind die hydrolysestabilen, polymerisierbaren Acrylphosphonsäuren der DE-A-1 974 670 8.

Ganz besonders bevorzugt sind ethylenisch ungesättigte Carbonsäuren der Formel: 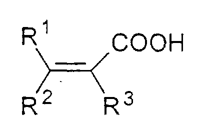 , in welcher bedeuten:
R¹, R²,R³ = H, C₁- bis C₂₅- Alkyl- oder -Cycloalkylreste, ggf. substituiert mit N, O, S, Si, P oder Halogen, oder aromatische C₆ bis C₁₂-Reste oder heterocyclische C₃ bis C₁₂-Reste, enthaltend N, O, S, P und ggf. substituiert mit Halogen,
sowie ethylenisch ungesättige Phosphorsäureester folgender Formel: 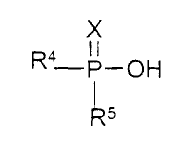 , in weicher bedeuten:
X = O, S;
R⁴ und R⁵ unabhängig voneinander H, OH, oder C₁ bis C₂₅-Alkyl oder -Cycloalkyl, ggf. substituiert oder verbrückt mit Heteroatomen wie N, Halogen, Si, O, oder S, aromatische C₆ bis C₁₂ -, oder/und heterocyclische C₄ bis C₁₂ -Reste, oder substiuiert mit Acrylsäureestern, wobei die Reste R⁴ und R⁵ auch unabhängig voneinander über O an den Phosphor gebunden sein können, oder 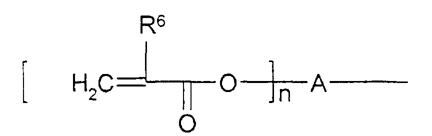 , wobei
R⁶ Wasserstoff oder C₁- bis C₆-Alkyl bedeutet,
n eine ganze Zahl ≥ 1 ist und,
A ein zweiwertiger C₁- bis C₂₅- Alkylen- oder Cycloalkylenrest, ggf. substituiert oder verbrückt mit N, O, S, Si, P oder Halogen, oder ein aromatischer C₆ bis C₁₂ - Rest, oder/und heterocyclischer C₄ bis C₁₂ -Rest mit N, O, S, oder P und ggf. substituiert mit Halogen, ist,
mit der Maßgabe dass die Gruppe, die den Rest R⁶ enthält, mindestens einmal vorhanden ist.

Typische Monomere bzw. Präpolymere, die nach dem Radikalkettenmechanismus aushärten und in Komponente ii) zusätzlich vorhanden sein können, sind Acrylate oder Methacrylate. Geeignet sind ein- und mehrfunktionelle (Meth)acrylatmonomere. Typische Vertreter dieser Verbindungs-klasse (siehe DE-A-4328 960) sind Alkyl(meth)acrylate, einschließlich der Cycloalkyl(meth)acrylate, Aralkyl(meth)acrylate und 2-Hydroxyalkyl(meth)acrylate, beispielsweise Hydroxypropylmethacrylat, Hydroxyethylmethacrylat, Isobornyl-acrylat, Isobornylethacrylat, Butylglycolmethacrylat, Acetylglykolmethacrylat, Triethylenglycolimethacrylat, Polyethylenglycoldimethacrylat, 2-Phenylethylethacrylat, 2-Ethylhexylethacrylat, Cyclohexylmethacrylat, Laurylmethacrylat und Hexandioldi(meth)acrylat.

Verwendet werden können auch langkettige Monomere, wie sie in der US-A-3 066 112 beschrieben sind, auf der Basis von Bisphenol-A und Glycidylmethacrylat oder deren durch Addition von Isocyanaten entstandene Derivate. Geeignet sind auch Verbindungen des Typs Bisphenyl-A-diethyloxy(meth)acrylat und Bisphenol-A-dipropyloxy(meth)acrylat. Weiterhin Verwendung finden können die oligoethoxylierten und oligopropoxylierten Bisphenol-A-diacryl- und -dimethacrylsäureester. Gut geeignet sind außerdem die in der DE-A-2 816 823 genannten Diacryl- und Dimethacrylsäureester des Bis(hydroxymethyl)-tricyclo[5.2.1.0^{2,6}]-decans und die Diacryl- und Dimethacryl-säureester der mit 1 bis 3 Ethylenoxidund/oder Propylenoxideinheiten verlängerten Verbindungen des Bis(hydroxymethyl)-tricyclo [5.2.1.0^{2,6}]-decans. Es können auch Gemische der genannten Monomeren verwendet werden.

Die erfindungsgemäßen Adhäsivsysteme können auch Füllstoffe, Farbstoffe, Fließmodifikatoren, Stabilisatoren, Lösungsmittel, ionenabgebende Substanzen, bakterizid oder antibiotisch wirksame Substanzen, die Röntgenopazität erhöhende Verbindungen oder weitere Modifikatoren enthalten.

Als Füllstoffe sind beispielsweise Stoffe geeignet, wie sie in üblichen Dentalmaterialien verwendet werden, besonders bevorzugt Quarz, Aerosile, hochdisperse Kieselsäuren, organische Füllstoffe oder Gläser, wie sie in marktüblichen Compositen verwendet werden oder Mischungen dieser Stoffe oder auch solche wie sie in der DE-A-196 48 283 A1 (Seite 10, Zeile 48-59) beschrieben sind.

Bei den ionenabgebenden Substanzen sind solche bevorzugt, die die Freisetzung von Fluoridionen ermöglichen, wie Fluoridsalze der ersten oder zweiten Hauptgruppe, wie Natriumfluorid oder Calciumfluorid, oder komplexe Fluoridsalze, wie KZnF₃, oder wie sie in der EP-A-0 717 977 beschrieben werden, Fluorid abgebende Gläser, sowie Mischungen dieser Fluroridionenquellen.

Als bakterizid oder antibiotisch wirksame Substanzen können beispielsweise Chlorhexidin, Pyridinumsalze oder die üblichen pharmazeutischen Substanzen, wie β-Lactamantibiotika (Penicilline), Cephalosporine, Tetracycline, Ansamycine, Kanamycine, Chloramphenicol, Fosfomycin, antibakterielle Makrolide, Polypeptid-Antibiotika, Chemotherapeutika, wie Sulfonamide, Dihydrofolatreduktase-Hemmstoffe, Nitrofuran-Derivate oder Gyrasehemmer, verwendet werden.

Die erfindungsgemäßen Adhäsivmischungen können auch Verdünnungsmittel enthalten, bevorzugt Lösungsmittel wie Dialkylketone (z. B. Aceton, Methylethylketon), Acetylaceton oder Alkohole (z. B. Ethanol, Propanol) oder auch dünnfließende polymerisierbare Substanzen wie 2-Hydroxyethylmethacrylat.

Als Stabilisatoren können beispielsweise Radikalfänger wie Hydroxybenzole, oder HALS (Hindered Amines Light Stabilizers) verwendet werden.

Außerdem kann zusätzlich mindestens eine nicht polymerisierbare Säure enthalten sein, wie eine ungesättigte Carbonsäure, Phosphorsäure, Phosphonsäure, Schwefelsäure, Sulfinsäure, Sulfensäure, Mineralsäure, Lewissäure oder Komplexsäure, wie beispielsweise H₂PtCl₆.

Enthalten die adhäsiven Mischungen über die Komponenten i) und ii) hinaus Zusatzstoffe, so können diese in den Mengen von 0,1 Gew.-% bis 85 Gew.-% einzeln oder gemischt vorhanden sein, wobei die Mischung so zubereitet werden, dass sie sich mit den Komponenten i) und ii) insgesamt zu 100 Gew.-% ergänzen.

Die adhäsive Mischung umfasst beispielsweise folgende Bestandteile:
i) ein radikalisches Initiatorsystem in einer Menge von 1 bis 5 Gew.-%.
ii) radikalisch polymerisierbare Monomere, die säurefunktionell sind oder Gruppen enthalten, die Säuren bilden können, in einer Menge von 3 bis 99 Gew.-%.
iii) radikalisch polymerisierbare Monomere, die nicht sauer sind, in einer Menge von 0 bis 90 Gew.-%.
iv) Lösungsmittel in einer Menge von 1 bis 75 Gew.-%.
v) ein reaktives Lösungsmittel in einer Menge von 1 bis 30 Gew.-%.
vi) Füllstoffe in einer Menge von 0 bis 75 Gew.-%.
vii) bakteriozid wirkende Substanzen bzw. Konservierungsmittel in einer Menge von 0 bis 20 Gew.-%.
viii) ionenabgebenden Substanzen in einer Menge von 0 bis 25 Gew.-%.
ix) Stabilisatoren in einer Menge von 0 bis 10 Gew.-%.
x) nicht polymerisierbare Säuren in einer Menge von 0 bis 25 Gew.-%.

Im folgenden wird die Erfindung anhand von Beispielen näher beschrieben.

### Haftmessung an Rinderzähnen durch adhäsive Befestigung eines Füllungsmaterials:

Die Überprüfung des Haftverbundes erfolgte durch einen Haftabzugsversuch an Rinderzähnen. Pro Versuch wurden 5 frisch extrahierte Rinderzähne mittels Schleifpapier soweit abgeschliffen, dass eine ausreichend große freiliegende Dentinfläche entstand. Auf diese Flächen wurden jeweils Wachsplättchen mit einem ausgestanzten Loch von 6 mm geklebt, um eine standardisierte Haftfläche zu erhalten. Die weitere Behandlung der Prüffläche und das Auftragen der Adäsivmischung erfolgte jeweils so, wie es bei den Herstellungsbeispielen angegeben ist.

### Herstellung eines kationisch härtenden Füllungsmaterials:

In einem Dreifingerkneter werden die folgenden Bestandteile zu einer homogenen Paste geknetet. Man verwendet auf 100 g Paste:
- 75,000 Gew.-% (75,000 g) Quarz (mittlere Korngröße 0,9 µm, wurde mit 5 Gew.-% Glycidyloxypropyltrimethoxysilan silanisiert);
- 0,525 Gew.-% (0,525 g) 4-Methylphenyl-4-isopropylphenyl-iodoniumtetrakis-(penta-fluorophenyl)borat;
- 0,223 Gew.-% (0,223 g) Campherchinon (Fa. Merck, Darmstadt);
- 0,001 Gew.-% (0,001 g) Ethyl-4-dimethylaminobenzoat (Fa. Merck, Darmstadt);
- 0,001 Gew.-% (0,001 g) 2-Butoxyethyl-4-dimethylaminobenzoat;
- 12,125 Gew.-% (12,125 g) 3,4-Epoxycyclohexyl-3,4-epoxycyclohexancarboxylat;
- 12,125 Gew.-% (12,125 g) 1,3,5,7-Tetrakis-(2,1-ethandiyl-3,4-epoxycyclohexyl)-1,3,5,7-tetramethylcyclotetrasiloxan.

### Herstellung der erfindungsgemäßen Adhäsivmischung 1:

Zur Herstellung von 10 g der Adhäsivmischung 1 werden die folgenden Bestandteile intensiv miteinander vermischt:
- 22,300 Gew.-% (2,930 g) (2-Hydroxyethyl)-methacrylat (Fa. Merck, Darmstadt);
- 7,000 Gew.-% (0,700 g) Ethanol
- 1,200 Gew.-% (0,120 g) Campherchinon (Fa. Merck, Darmstadt);
- 0,900 Gew.-% (0,090 g) Ethyl-4-dimethylaminobenzoat (Fa. Merck, Darmstadt);
- 68,600 Gew.-% (6,860 g) 4-Methacryloxyethyltrimellitsäure (siehe US 4 148 988).

### Herstellung der erfindungsgemäßen Adhäsivmischung 2:

Zur Herstellung von 10 g der Adhäsivmischung 2 werden die folgenden Bestandteile intensiv miteinander vermischt:
- 19,600 Gew.-% (1,960 g) 2-Hydroxyethylmethacrylat (Fa. Merck, Darmstadt);
- 1,200 Gew.-% (0,120 g) Campherchinon (Fa. Merck, Darmstadt);
- 0,900 Gew.-% (0,090 g) Ethyl-4-dimethylaminobenzoat (Fa. Merck, Darmstadt);
- 68,300 Gew.-% (6,830 g) Methacryloyl-oxydecyl-phosphat.
- 10,000 Gew.-% (1,000 g) Wasser.

### Herstellung der erfindungsgemäßen Adhäsivmischung 3:

Zur Herstellung von 10 g der Adhäsivmischung 1 werden die folgenden Bestandteile intensiv miteinander vermischt:
- 5,500 Gew.-% (0,550 g) (2-Hydroxyethyl)-methacrylat (Fa. Merck, Darmstadt);
- 1,200 Gew.-% (0,120 g) Campherchinon (Fa. Merck, Darmstadt);
- 0,900 Gew.-% (0,090 g) Ethyl-4-dimethylaminobenzoat (Fa. Merck, Darmstadt);
- 25,000 Gew.-% (2,500 g) 4-Methacryloxyethyltrimellitsäure (siehe US-A-4,148,988).
- 55,000 Gew.-% (5,500 g) Methacryloyl-oxydecyl-phosphat.
- 12,400 Gew.-% (1,240 g) Wasser.

### Herstellung der Vergleichsmischung 1:

Zur Herstellung von 10 g der Vergleichsmischung 1 werden die folgenden Bestandteile intensiv miteinander vermischt:
- 97,300 Gew.-% (9,730 g) 1,3,5,7-Tetrakis-(2,1-ethandiyl-3,4-epoxycyclohexyl)-1,3,5,7-tetramethylcyclotetrasiloxan;
- 2,0 Gew.-% (0,200 g) Rhodorsil PI 2074 (Fa. Rhone Poulenc, lodoniumsalz);
- 0,500 Gew.-% (0,050 g) Campherchinon (Fa. Merck, Darmstadt);
- 0,200 Gew.-% (0,020 g) BEDB (Fa. Lambson);

### Herstellung der Vergleichsmischung 2:

Zur Herstellung von 10 g der Vergleichsmischung 2 werden die folgenden Bestandteile intensiv miteinander vermischt:
- 97,300 Gew.-% (9,730 g) 3,4-Epoxycyclohexyl-3,4-epoxycyclohexancarboxylat;
- 2,0 Gew.-% (0,200 g) Rhodorsil PI 2074 (Fa. Rhone Poulenc, lodoniumsalz);
- 0,500 Gew.-% (0,050 g) Campherchinon (Fa. Merck, Darmstadt);
- 0,200 Gew.-% (0,020 g) BEDB (Fa. Lambson);

### Herstellung der Vergleichsmischung 3:

Zur Herstellung von 10 g der Vergleichsmischung 3 werden die folgenden Bestandteile intensiv miteinander vermischt:
- 48,650 Gew.-% (4,865 g) 3,4-Epoxycyclohexylmethyl-3,4-epoxycyclohexancarboxylat;
- 48,650 Gew.-% (4,865 g) 1,3,5,7-Tetrakis-(2,1-ethandiyl-3,4-epoxycyclohexyl)-1,3,5,7-tetramethylcyclotetrasiloxan;
- 2,000 Gew.-% (0,200 g) Rhodorsil PI 2074 (Fa. Rhone Poulenc, lodoniumsalz);
- 0,500 Gew.-% (0,050 g) Campherchinon (Fa. Merck, Darmstadt);
- 0,200 Gew.-% (0,020 g) BEDB (Fa. Lambson);

### Vorgehensweise:

Die Prüffläche wurde in Anlehnung an das praxisübliche Vorgehen mittels einer üblichen Phosphorsäurelösung (Ätzgel Minitip®, Fa. ESPE Dental AG, Seefeld) für 20 Sekunden angeätzt und anschließend mit Wasser abgespült. Auf die derart vorbereiteten Dentinflächen wurde eine zur vollständigen Benetzung der Prüfoberfläche ausreichende Menge der Versuchsmischungen mit einem Microbrush 20 sec. lang eingearbeitet, kurz mit Druckluft verblasen und mittels eines Lichtpolymerisationsgerätes (Elipar Highlight®, Fa. ESPE) für 20 Sekunden polymerisiert. Anschließend wurde das kationisch härtende Füllungsmaterial, dessen Herstellung oben beschrieben ist, die Aussparungen der Wachsplättchen eingebracht und durch 40 sec. Belichtung auspolymerisiert. Das Wachsplättchen wurde entfernt und die Prüfkörper 24 h bei 36°C und 100% Luftfeuchtigkeit gelagert. Dann wurden die Prüfkörper in einem Zugversuch (Zwick Universalprüfmaschine) abgezogen.

Die dabei ermittelten Haftwerte sind Tabelle 1 zu entnehmen.

**Tabelle 1:**

| Haftung der in den Beispielen beschriebenen Adhäsivmischungen: | |
|---|---|
| Adhäsivmischung | Dentinhaftung [MPa]* |
| Adhäsivmischung 1 | 3,6 |
| Adhäsivmischung 2 | 4,2 |
| Adhäsivmischung 3 | 3,8 |
| Pertac Universal Bond (Fa. ESPE) | 2,2 |
| Prime & Bond NT (Fa. Dentsply) | 1,9 |
| Scotchbond Multi Purpose Plus (3M) | 2,1 |
| Visio Bond (Fa. ESPE) | 0,0 |
| Vergleichsmischung 1 | 0,0 |
| Vergleichsmischung 2 | 0,0 |
| Vergleichsmischung 3 | 0,0 |

| | |
|---|---|
| * Mittelwert aus je 5 Messungen | |

Obige Tabelle zeigt, dass mit den kationisch polymerisierenden Vergleichsmischungen 1 bis 3 keine Haftung auf der Wasser enthaltenden Zahnhartsubstanz erzielt werden kann. Andererseits wird gezeigt, das mit den erfindungsgemäßen, radikalisch polymerisierenden Adhäsivmischungen 1 bis 3 und auch mit den aufgezeigten, erfindungsgemäßen, radikalisch polymerisierenden käuflich erwerbbaren Adhäsivsystemen, kationisch polymerisierbare Dentalmaterialien auf gegebener Substanz adhäsiv befestigt werden können.

## Patentansprüche

1. Verwendung einer Zusammensetzung, die radikalisch polymerisierbar ist und 1 bis 30 Gew.-% mindestens eines reaktiven Lösungsmittels mit einem pKS-Wert kleiner oder gleich dem von Aceton enthält, wobei die Zusammensetzung mindestens eine Komponente i), die befähigt ist, eine radikalische Reaktion zu starten, und mindestens eine Komponente ii), die 3 bis 100 Gew.-% radikalisch polymerisierbare Monomere enthält, die säurefunktionell sind oder Gruppen enthalten, die Säuren bilden können, enthält, zur Herstellung eines Adhäsivsystems zur Befestigung von Materialien, die nur oder auch kationisch polymerisierbar sind, auf Wasser enthaltendem Hartgewebe.

2. Verwendung nach Anspruch 1, wobei das reaktive Lösungsmittel Hydroxylgruppen trägt.

3. Verwendung nach einem der vorstehenden Ansprüche, wobei die Komponente i) in Anteilen von 0,01 bis 10 Gew.-%, und die Komponente ii) in Anteilen von 90,00 bis 99,99 Gew.-% vorliegt.

4. Verwendung nach einem der vorstehenden Ansprüche, wobei die Materialien Dentalmaterialien sind und das Hartgewebe Zahn ist.

5. Verwendung einer Mischung nach einem der vorstehenden Ansprüche, wobei das Adhäsivsystem als Komponente ii) eine einfach oder mehrfach ethylenisch ungesättigte organische Säure oder deren Anhydrid oder deren Säurechlorid enthält.

6. Verwendung einer Mischung nach einem der vorstehenden Ansprüche, wobei zusätzlich mindestens eine der folgenden Komponenten oder eine Mischung der folgenden Komponenten enthalten sind: iii) ein weiterer radikalischer und/oder kationischer Polymerisationsinitiator; iv) ein Verdünnungsmittel, vorzugsweise ein in Adhäsivmischungen übliches organisches Lösungsmittel oder eine dünnfließende Verbindung, die radikalisch polymerisierbare Gruppen enthält; v) ein Füllstoff, wie er in üblichen Dentalmaterialien verwendet wird; vi) eine Fluoridionenquelle; vii) eine Säure, die keine Doppelbindung enthält; viii) ein bakterizid wirkendes Mittel oder übliches Antibiotika.

7. Verwendung einer Mischung nach einem der vorstehenden Ansprüche, wobei die Mischung durch Zufuhr von elektromagnetischer Strahlung polymerisiert wird, vorzugsweise durch Bestrahlung mit Licht der Wellenlänge von 350 bis 1000 nm.

8. Verwendung einer Mischung nach einem der vorstehenden Ansprüche, wobei die Komponente ii) eine ethylenisch ungesättigte Carbonsäure der folgenden Formel enthält: 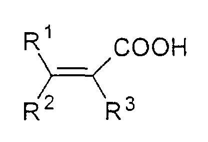 in welcher bedeuten:
R¹, R²,R³ = H, C₁- bis C₂₅- Alkyl- oder -Cycloalkylreste, ggf. substituiert mit N, O, S, Si, P oder Halogen, oder aromatische C₆ bis C₁₂-Reste oder heterocyclische C₃ bis C₁₂-Reste mit N, O, S, P und ggf. substituiert mit Halogen,
oder einen ethylenisch ungesättigten Phosphorsäureester der folgenden Formel enthält: 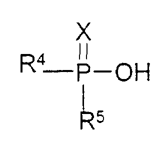 , in welcher bedeuten:
X = O, S;
R⁴ und R⁵ unabhängig voneinander H, OH, oder C₁ bis C₂₅-Alkyl oder -Cycloalkyl, ggf. substituiert oder verbrückt mit Heteroatomen, wie N, Halogen, Si, O, oder S, aromatische C₆ bis C₁₂ -, oder/und heterocyclische C₄ bis C₁₂ -Reste oder substiuiert mit Acrylsäureestern, wobei die Reste R⁴ und R⁵ auch unabhängig voneinander über O an den Phosphor gebunden sein können, oder 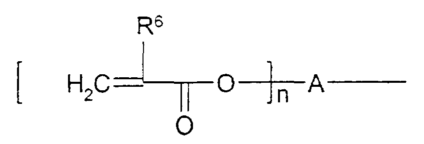 , wobei
R⁶ Wasserstoff oder C₁- bis C₆-Alkyl bedeutet,
n eine ganze Zahl ≥ 1 ist und,
A ein zweiwertiger C₁- bis C₂₅- Alkylen- oder Cycloalkylenrest ggf. substituiert oder verbrückt mit N, O, S, Si, P oder Halogen oder ein aromatischer C₆ bis C₁₂ - Rest, oder/und heterocyclischer C₄ bis C₁₂ -Rest mit N, O, S, oder P und ggf. substituiert mit Halogen, ist, mit der Maßgabe, dass der R⁶ enthaltene Rest mindestens einmal vorhanden ist.

9. Verwendung einer Mischung nach einer der vorstehenden Ansprüche, wobei unmittelbar nach dem Aufbringen der Mischung auf die Zahnhartsubstanz mit nur oder auch kationisch polymerisierbarem Material überschichtet wird.

10. Kit, umfassend a) ein Adhäsivsystem, enthaltend eine Komponente i), die befähigt ist, eine radikalische Reaktion zu starten und eine Komponente ii), die radikalische polymerisierbare Monomere enthält, die säurefunktionell sind oder Gruppen enthalten, die Säuren bilden können, iii) 1 bis 30 Gew.-% eines reaktiven Lösungsmittels mit einem pKS-Werte kleiner oder gleich dem von Aceton und b) ein Material, das nur oder auch kationisch polymerisierbar ist.

11. Kit nach Anspruch 10, wobei die Komponente iii) von den übrigen Komponenten getrennt gelagert ist.

## Claims

1. Use of a composition which is free-radically polymerizable and contains from 1 to 30% by weight of at least one reactive solvent having a pKa value less than or equal to that of acetone, the composition comprising at least one component i) capable of initiating a free-radical reaction and at least one component ii) containing from 3 to 100% by weight of free-radically polymerizable monomers which are acid-functional or contain groups that are able to form acids for producing an adhesive system for affixing materials which are cationically polymerizable or which can only be polymerized cationically to water-containing hard tissue.

2. Use according to Claim 1, wherein the reactive solvent carries hydroxyl groups.

3. Use according to one of the above claims, wherein component i) is present in fractions of from 0.01 to 10% by weight and component ii) in fractions of from 90.00 to 99.99% by weight.

4. Use according to one of the above claims, the materials being dental materials and the hard tissue being tooth.

5. Use of a mixture according to one of the above claims, wherein the adhesive system comprises as component ii) a mono- or polyethylenically unsaturated organic acid or anhydride thereof or acid chloride thereof.

6. Use of a mixture according to one of the above claims, wherein at least one of the following components or a mixture of the following components are additionally present: iii) a further free-radical and/or cationic polymerization initiator; iv) a diluent, preferably an organic solvent which is customary in adhesive mixtures or a highly mobile compound containing free-radically polymerizable groups; v) a filler such as is used in customary dental materials; vi) a fluoride ion source; vii) an acid containing no double bond; viii) a bactericidal agent or customary antibiotic(s).

7. Use of a mixture according to one of the above claims, wherein the mixture is polymerized by supplying electromagnetic radiation, preferably by irradiation with light of wavelength from 350 to 1000 nm.

8. Use of a mixture according to one of the above claims, wherein component ii) comprises an ethylenically unsaturated carboxylic acid of the following formula: 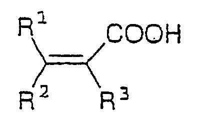 in which:
R¹, R², R³ = H, C₁ to C₂₅ alkyl or cycloalkyl radicals, unsubstituted or substituted by N, O, S, Si, P or halogen, or aromatic C₆ to C₁₂ radicals or heterocyclic C₃ to C₁₂ radicals containing N, O, S, P and unsubstituted or substituted by halogen,
or an ethylenically unsaturated phosphoric ester of the following formula: 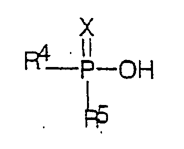 in which:
X = O, S;
R⁴ and R⁵ independently of one another are H, OH, or C₁ to C₂₅ alkyl or cycloalkyl, unsubstituted or unbridged or substituted or bridged by heteroatoms such as N, halogen, Si, O or S, aromatic C₆ to C₁₂ radicals and/or heterocyclic C₄ to C₁₂ radicals, or substituted by acrylic esters, it also being possible for the radicals R⁴ and R⁵ independently of one another to be attached to the phosphorus via O, or 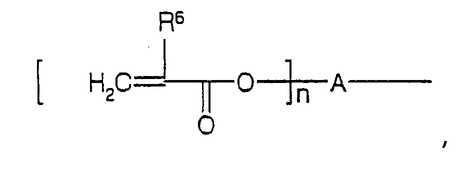 where
R⁶ is hydrogen or C₁ to C₆ alkyl
n is an integer ≥ 1, and
A is a divalent C₁ to C₂₅ alkylene or cycloalkylene radical, unsubstituted or unbridged or substituted or bridged by N, O, S, Si, P or halogen, or an aromatic C₆ to C₁₂ radical or heterocyclic C₄ to C₁₂ radical containing N, O, S, or P and unsubstituted or substituted by halogen, with the proviso that the radical that contains R⁶ is present at least once.

9. Use of a mixture according to one of the above claims, wherein directly following the application of the mixture to the hard tooth substance overcoating is carried out with material which is cationically polymerizable or which can only be polymerized cationically.

10. Kit comprising a) an adhesive system comprising a component i) capable of initiating a free-radical reaction and a component ii) which comprises free-radically polymerizable monomers which are acid-functional or contain groups that are able to form acids, iii) from 1 to 30% by weight of a reactive solvent having pKa less than or equal to that of acetone and b) a material which is cationically polymerizable or which can only be polymerized cationically.

11. Kit according to Claim 10, wherein component iii) is stored separately from the other components.

## Revendications

1. Utilisation d'une composition qui est polymérisable par voie radicalaire et comprend de 1 à 30 % en poids d'au moins un solvant réactif présentant un pKS inférieur ou égal à celui de l'acétone, la composition comprenant au moins un composant i) capable d'amorcer une réaction radicalaire et au moins un composé ii) comprenant de 3 à 100 % en poids de monomères polymérisables par voie radicalaire, qui sont à fonction acide ou renferment des groupes qui peuvent former des acides, pour la production d'un système adhésif destiné à la fixation de matières qui sont polymérisables uniquement ou également par voie cationique, sur un tissu dur contenant de l'eau.

2. Utilisation selon la revendication 1, dans laquelle les solvants réactifs portent des groupes hydroxy.

3. Utilisation selon l'une quelconque des revendications précédentes, dans laquelle le composant i) est présent en proportion de 0,01 à 10 % en poids et le composant ii) en proportion de 90,00 à 99,99 % en poids.

4. Utilisation selon l'une quelconque des revendications précédentes, dans laquelle les matières sont des matières dentaires et le tissu dur est une dent.

5. Utilisation d'un mélange selon l'une quelconque des revendications précédentes, dans laquelle le système adhésif comprend, en tant que composant ii), un acide organique mono- ou polyéthyléniquement insaturé ou son anhydride ou son chlorure d'acide.

6. Utilisation d'un mélange selon l'une quelconque des revendications précédentes, dans laquelle en outre au moins l'un des composants suivants ou un mélange des composants suivants sont présents : iii) un amorceur de polymérisation radicalaire et/ou cationique supplémentaire ; iv) un diluant, de préférence un solvant organique habituel dans des mélanges adhésifs ou un composé fluidifiant, qui renferme des groupes polymérisables par voie radicalaire ; v) une charge, telle qu'elle est utilisée dans des matières dentaires habituelles ; vi) une source d'ions fluorure ; vii) un acide qui ne renferme pas de double liaison ; viii) un agent à action bactéricide ou un antibiotique habituel.

7. Utilisation d'un mélange selon l'une quelconque des revendications précédentes, dans laquelle le mélange est polymérisé par apport d'un rayonnement électromagnétique, de préférence par irradiation avec de la. lumière présentant une longueur, d'onde de 350 à 1000 nm.

8. Utilisation d'un mélange selon l'une quelconque des revendications précédentes, dans laquelle le composant ii) renferme un acide carboxylique éthyléniquement insaturé de formule suivante : 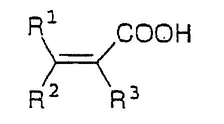 dans laquelle :
R¹, R² et R³ représentent H, des radicaux alkyle ou cycloalkyle en C₁ à C₂₅, éventuellement substitués par N, O, S, Si, P ou un halogène, ou des radicaux aromatiques en C₆ à C₁₂ ou des radicaux hétérocycliques en C₃ à C₁₂ renfermant N, O, S, P et éventuellement substitués par un halogène,
ou un ester d'acide phosphorique éthyléniquement insaturé de formule suivante : 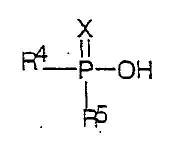 dans laquelle :
X représente O, S ;
R⁴ et R⁵ représentent, indépendamment l'un de l'autre, H, OH, ou un alkyle ou cycloalkyle en C₁ à C₂₅ éventuellement substitué ou ponté par des hétéroatomes, tels que N, un halogène, Si, O ou S, des radicaux aromatiques en C₆ à C₁₂ et/ou des radicaux hétérocycliques en C₄ à C₁₂, ou substitué par des esters d'acide acrylique, les radicaux R⁴ et R⁵ pouvant également, indépendamment l'un de l'autre, être liés au phosphore par l'intermédiaire de O, ou 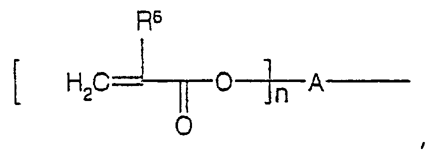
dans laquelle
R⁶ représente un hydrogène ou un alkyle en C₁ à C₆,
n représente un entier ≥ 1, et
A représente un radical alkyle ou cycloalkyle en C₁ à C₂₅ bivalent, éventuellement substitué ou ponté par N, O, S, Si, P ou un halogène ou un radical aromatique en C₆ à C₁₂ et/ou un radical hétérocyclique en C₄ à C₁₂ renfermant N, O, S ou P et éventuellement substitué par un halogène, à condition que le radical renfermant R⁶ soit présent au moins une fois.

9. Utilisation d'un mélange selon l'une quelconque des revendications précédentes, dans laquelle immédiatement après l'application du mélange sur la substance dure dentaire, on procède au revêtement avec une matière polymérisable uniquement ou également par voie cationique.

10. Kit comportant a) un système adhésif comprenant un composant i) capable d'amorcer une réaction radicalaire et un composant ii) comprenant des monomères polymérisables par voie radicalaire qui sont à fonction acide ou renferment des groupes qui peuvent former des acides, iii) de 1 à 30 % en poids d'un solvant réactif présentant un pKS inférieur ou égal à celui de l'acétone et b) une matière qui est polymérisable uniquement ou également par voie cationique.

11. Kit selon la revendication 10, dans lequel le composant iii) est stocké séparément des autres composants.
